# EUROPÄISCHE PATENTSCHRIFT

(11) **EP 3 226 918 B1**
(45) Veröffentlichungstag und Bekanntmachung des Hinweises auf die Patenterteilung: **07.06.2023**
(21) Anmeldenummer: 15805410.6
(22) Anmeldetag: 23.11.2015
(51) Int. Cl.: A61L 2/24, A61B 1/12, A61L 2/18

(54) **AUFBEREITUNGSSYSTEM ZUM REINIGEN UND/ODER DESINFIZIEREN VON MEDIZINISCHEN GERÄTEN UND VERFAHREN ZUM BETREIBEN DESSELBEN**
PREPARATION SYSTEM FOR CLEANING AND/OR DISINFECTING MEDICAL DEVICES, AND METHOD FOR OPERATING SAME
SYSTÈME DE TRAITEMENT POUR LE NETTOYAGE ET/OU LA DÉSINFECTION D'APPAREILS MEDICAUX ET PROCÉDÉ DE FONCTIONNEMENT

(30) Priorität: 04.12.2014 DE 102014224843
(43) Veröffentlichungstag der Anmeldung: 11.10.2017
(73) Patentinhaber: Olympus Winter&Ibe GmbH, 22045 Hamburg (DE)
(72) Erfinder: OTTENS, Benjamin, 22309 Hamburg (DE)
(74) Vertreter: Seemann & Partner Patentanwälte mbB
(86) Internationale Anmeldenummer: PCT/EP2015/077327
(87) Internationale Veröffentlichungsnummer: WO 2016/087244

(56) Entgegenhaltungen:
- WO-A1-2012/010902
- JP-A- 2009 172 228
- JP-B2- 4 084 336
- US-A1- 2008 190 460
- US-A1- 2010 266 447
- US-B2- 8 123 871

## Beschreibung

Die Erfindung betrifft ein Aufbereitungssystem zum Reinigen und/oder Desinfizieren von medizinischen Geräten, insbesondere Endoskopen, umfassend zumindest ein Aufbereitungsgerät, wobei ein Aufbereitungsraum des Aufbereitungsgeräts dazu vorgesehen ist, dass zumindest ein, insbesondere in dem Reinigungskorb angeordnetes medizinisches Gerät in einem Reinigungs- und/oder Desinfiziervorgang innerhalb des Aufbereitungsraums gereinigt und/oder desinfiziert wird, und wobei der Aufbereitungsraum durch eine an einer Gerätefront des Aufbereitungsgeräts vorhandene Tür geschlossen oder schließbar ist. Ferner betrifft die Erfindung ein Verfahren zum Betreiben eines solchen Aufbereitungssystems.

An die Aufbereitung, d.h. die Reinigung und/oder Desinfektion, von chirurgischen Instrumenten, beispielsweise Endoskopen, werden hohe Anforderungen gestellt. Nach der Benutzung des chirurgischen Instruments wird dieses in einem Aufbereitungsgerät, d.h. einer Reinigungs- und/oder Desinfiziereinrichtung, gereinigt und/oder desinfiziert und so für die nächste Verwendung vorbereitet. Ein solches Aufbereitungsgerät für Endoskope ist beispielsweise unter der Bezeichnung ETD (für Endo Thermo Desinfector) des Herstellers Olympus Medical Systems bekannt. Bei einigen Aufbereitungsgeräten wird eine räumliche Trennung zwischen der unreinen und der reinen Seite vorgenommen. Ein solches Aufbereitungsgerät umfasst zwei vielfach elektrisch angetriebene Türen, die den Behandlungsraum an einander gegenüberliegenden Seiten schließen. Die Türen des Aufbereitungsgeräts werden durch Schalter in der Maschinenfront oder durch einen in der Nähe des Geräts platzierten Fußschalter betätigt. Ein solches Gerät ist beispielsweise unter der Bezeichnung ETD Double der Anmelderin bekannt.

Zu reinigende bzw. zu desinfizierende Endoskope werden, bevor sie der Aufbereitung zugeführt werden, in einen Reinigungskorb eingelegt und an dessen Adapter angeschlossen. Ein voll bestückter Reinigungskorb wiegt einschließlich des eingelegten Endoskops und der Adapter einige Kilogramm. Eine mit der Aufbereitung betraute Person hält den voll bestückten Reinigungskorb in den Händen, während sie den Fußschalter betätigt, um die Tür des Aufbereitungsgeräts zu öffnen. Da während des Öffnungsvorgangs unvermeidbar eine gewisse Zeitspanne vergeht, ist die Person gezwungen, für diese Zeit den voll bestückten und schweren Reinigungskorb in den Händen zu halten, bevor sie ihn in den Aufbereitungsraum des Aufbereitungsgeräts einschieben kann. Dieser Vorgang bedeutet eine nicht zu vernachlässigende körperliche Belastung. Ferner wird der Arbeitsablauf unnötig unterbrochen und der mögliche Durchsatz des Aufbereitungsgeräts nicht optimal ausgeschöpft.

Aus der WO 2012/010902 A1 ist eine Vorrichtung zum Reinigen und Desinfizieren eines Stethoskops bekannt. Diese umfasst eine austauschbare Kartusche, die ein Reinigungsmedium zur Reinigung des medizinischen Geräts umfasst. In der Vorrichtung ist eine Öffnung vorhanden, durch die das Stethoskop in Kontakt mit einer Oberfläche gebracht werden kann, die das Stethoskop reinigt. Die Vorrichtung umfasst einen IR-Sensor, der die Gegenwart des Stethoskops feststellt. In Reaktion auf einen Messwert des IR-Sensors wird ein Verschluss der genannten Öffnung geöffnet und der Reinigungsvorgang in Gang gesetzt.

JP 4084336 B2 zeigt eine Sterilisationsvorrichtung zum Desinfizieren eines Handstücks zur oralen Behandlung eines Patienten. Das Handstück wird in eine transparente Röhre eingelegt, um es mittels ultravioletter Strahlung zu desinfizieren. Um zu verhindern, dass ein noch nicht sterilisiertes Handstück eine Tür der Vorrichtung berührt, ist ein Sensor mit einer Lichtquelle und einem Lichtdetektor vorgesehen, welcher die Tür automatisch öffnet, wenn der Lichtdetektor eine Abnahme der Lichtintensität detektiert.

Es ist eine Aufgabe der Erfindung, ein Aufbereitungssystem zum Reinigen und/oder Desinfizieren von medizinischen Geräten sowie ein Verfahren zum Betreiben eines solchen Aufbereitungssystems anzugeben, wobei die Ergonomie des Aufbereitungssystems verbessert und der Durchsatz erhöht werden soll.

Die Aufgabe wird gelöst durch ein Aufbereitungssystem zum Reinigen und/oder Desinfizieren von medizinischen Geräten, insbesondere Endoskopen, umfassend zumindest ein Aufbereitungsgerät, wobei ein Aufbereitungsraum des Aufbereitungsgeräts dazu vorgesehen ist, dass zumindest ein, insbesondere in dem Reinigungskorb angeordnetes medizinisches Gerät in einem Reinigungs- und/oder Desinfiziervorgang innerhalb des Aufbereitungsraums gereinigt und/oder desinfiziert wird, und wobei der Aufbereitungsraum durch eine an einer Gerätefront des Aufbereitungsgeräts vorhandene Tür geschlossen oder schließbar ist, wobei das Aufbereitungssystem dadurch fortgebildet ist, dass das medizinische Gerät und/oder der Reinigungskorb ein maschinenlesbares Kennzeichnungsmerkmal umfassen und das Aufbereitungsgerät einen berührungslos arbeitenden Sensor umfasst, der dazu eingerichtet ist mit dem maschinenlesbaren Kennzeichnungsmerkmal zusammenzuwirken, wobei der Sensor ferner dazu eingerichtet ist, zeitabhängig Ortskoordinaten des medizinischen Geräts und/oder des Reinigungskorbs zu erfassen und an eine Steuereinrichtung des Aufbereitungsgeräts auszugeben, wobei die Steuereinrichtung dazu eingerichtet ist, ausgehend von den zeitabhängigen Ortskoordinaten, eine Bewegungsanalyse des erfassten medizinischen Geräts und/oder Reinigungskorbs vorzunehmen und festzustellen, ob sich das medizinische Gerät und/oder der Reinigungskorb auf die in der Gerätefront vorhandene Tür des Aufbereitungsgeräts zubewegen und sofern dies festgestellt wird, ein Türöffnungssignal zu generieren und den Zugang zu dem Behandlungsraum durch Öffnen der Tür zu ermöglichen, wobei die Steuereinrichtung dazu eingerichtet ist, aus den zeitabhängigen Ortskoordinaten eine Raumkurve des medizinischen Geräts und/oder des Reinigungskorbs zu bestimmen und sofern diese Raumkurve für zumindest eine vorgegebene Wegstrecke und/oder zumindest eine vorgegebene Zeitspanne und/oder mit zumindest einer vorgegebenen Geschwindigkeit in Richtung der in der Gerätefront des Aufbereitungsgeräts vorhandenen Tür verläuft, dies als Hinweis darauf zu werten, dass sich das medizinische Gerät und/oder der Reinigungskorb auf die Tür des Aufbereitungsgeräts zubewegen.

Insbesondere ist der Aufbereitungsraum des Aufbereitungsgeräts dazu vorgesehen, dass das medizinische Gerät in einem Reinigungs- und/oder Desinfiziervorgang reinigbar und/oder desinfizierbar ist.

Die Erfindung beruht auf der Erkenntnis, dass die Wartezeit vor dem Aufbereitungsgerät und die damit verbundene körperliche Belastung für die Reinigungsperson erheblich reduziert werden, wenn das Aufbereitungsgerät bereits beginnt, die Tür zu öffnen, bevor die mit der Aufbereitung der medizinischen Geräte betraute Person direkt vor dem Aufbereitungsgerät steht.

In vielen Fällen dauert der Öffnungsvorgang der Tür ca. 20 Sekunden. Bei einem Gewicht des voll beladenen Reinigungskorbs von in etwa 8 kg, stellt dies eine nicht unerhebliche physische Belastung dar. Die Betätigung eines Fußschalters ist schwierig, wenn gleichzeitig der voll beladene Reinigungskorb in den Händen gehalten werden muss. Schließlich ist der Blick auf den Bodenbereich, in dem der Fußschalter angeordnet ist, durch den in den Händen gehaltenen Reinigungskorb verdeckt. Es ist vielfach nötig, den Fußschalter "blind" zu suchen. Dieser Vorgang stellt eine zusätzliche körperliche Belastung dar und ist ein potenziell unfallträchtiger Bewegungsvorgang.

Ferner vergeht auch bei dieser Tätigkeit unnötig Zeit, sowohl Arbeitszeit der Reinigungsperson als auch Maschinenzeit des Aufbereitungsgeräts. Wird hingegen - wie erfindungsgemäß vorgesehen - die Tür des Aufbereitungsgeräts bereits geöffnet, wenn sich die mit der Aufbereitung betraute Person der in der Gehäusefront vorhandenen Tür des Aufbereitungsgeräts nähert, wird die Wartezeit vor der Maschine deutlich reduziert, was insbesondere bei großen Aufbereitungsräumen einen erheblichen Zeitvorteil darstellt.

Neben der ergonomischen Optimierung des Aufbereitungssystems wird also nicht unnötig Maschinenzeit des Aufbereitungsgeräts verschwendet, wodurch dessen Auslastung verbessert ist. Der Workflow innerhalb des Aufbereitungssystems ist optimiert.

Das Aufbereitungssystem ist derart eingerichtet, dass die Steuereinrichtung dazu eingerichtet ist, aus den zeitabhängigen Ortskoordinaten eine Raumkurve des medizinischen Geräts und/oder des Reinigungskorbs zu bestimmen und sofern diese Raumkurve für zumindest eine vorgegebene Wegstrecke und/oder zumindest eine vorgegebene Zeitspanne und/oder mit zumindest einer vorgegebenen Geschwindigkeit in Richtung der in der Gerätefront des Aufbereitungsgeräts vorhandenen Tür verläuft, dies als Hinweis darauf zu werten, dass sich das medizinische Gerät und/oder der Reinigungskorb auf die Tür des Aufbereitungsgeräts zubewegen.

Als Entscheidungskriterium, ob sich das medizinische Gerät und/oder der Reinigungskorb auf die in der Gerätefront vorhandene Tür zubewegen, wird eine Form der aus den zeitabhängigen Ortskoordinaten zusammengesetzten Raumkurve herangezogen. Diese Raumkurve, bei der es sich um eine Bewegungsbahn bzw. um eine Trajektorie des medizinischen Geräts oder des Reinigungskorbs, genauer des maschinenlesbaren Kennzeichnungsmerkmals dieser Einheiten handelt, umfasst eine Vielzahl von Ortskoordinaten. Diese werden beispielsweise in regelmäßigen zeitlichen Abständen erfasst oder weisen einen Zeitstempel auf. So ist es möglich, aus dem Differenzvektor zwischen aufeinanderfolgenden Ortskoordinaten eine Bewegungsrichtung und eine Bewegungsgeschwindigkeit zu ermitteln.

Es wird der jeweils aktuellste Abschnitt der Raumkurve von der Steuereinrichtung analysiert. Im Hinblick auf diese zurückgelegte Wegstrecke wird die Entscheidung getroffen, ob einer der genannten Grenzwerte überschritten ist, sich das medizinische Gerät und/oder der Reinigungskorb also für zumindest die vorgegebene Wegstrecke, Zeitspanne oder mit zumindest der vorgegebenen Geschwindigkeit auf die Gehäusefront des Aufbereitungsgeräts zubewegen.

Gemäß einer weiteren Ausführungsform ist das Aufbereitungssystem dadurch fortgebildet, dass die Steuereinrichtung dazu eingerichtet ist, anhand der zeitabhängigen Ortskoordinaten festzustellen, ob sich das medizinische Geräts und/oder der Reinigungskorb innerhalb eines Ladebereichs befinden, der an die die Tür umfassende Gerätefront angrenzt, und sofern dies der Fall ist, dies als Hinweis darauf zu werten, dass sich das medizinische Gerät und/oder der Reinigungskorb auf die Tür des Aufbereitungsgeräts zubewegen.

Neben einer Analyse von der Wegstrecke, Geschwindigkeit und Richtung einer Raumkurve ist es alternativ oder zusätzlich möglich, den Ladebereich zu definieren. Bei Eintritt des medizinischen Geräts oder des Reinigungskorbs öffnet sich die Tür des Aufbereitungsgeräts automatisch. Mit anderen Worten wird die Tür des Aufbereitungsgeräts geöffnet, sobald sich der Reinigungskorb bzw. das medizinische Geräte bis auf eine vorgegebene Distanz der Gerätefront nähert.

Im Vergleich zu einer Analyse der Raumkurve stellt diese Ausführungsform geringere Anforderungen an Logik und Rechenleistung der Steuereinrichtung. Eine solche Lösung ist daher vergleichsweise einfach und kostengünstig zu implementieren.

In einer weiteren Ausführungsform ist vorteilhaft vorgesehen, dass ausschließlich der Reinigungskorb zur Aufnahme des zumindest einen medizinischen Geräts mit dem maschinenlesbaren Kennzeichnungsmerkmal versehen ist.

Im klinischen Alltag wird eine Vielzahl verschiedener medizinischer Geräte verwendet, welche nicht durchgängig mit maschinenlesbaren Kennzeichnungsmerkmalen versehen sind. In einem Aufbereitungssystem, welches mit maschinenlesbaren Kennzeichnungsmerkmalen versehene Reinigungskörbe umfasst, ist es vorteilhaft möglich, sowohl medizinische Geräte mit als auch ohne maschinenlesbare Kennzeichnungsmerkmale aufzubereiten. Das Aufbereitungssystem ist besonders flexibel.

Gemäß weiterer vorteilhafter Ausführungsformen ist vorgesehen, dass das maschinenlesbare Kennzeichnungsmerkmal eine äußere Form des medizinischen Geräts und/oder des Reinigungskorbs ist und/oder das maschinenlesbare Kennzeichnungsmerkmal ein auf einer Außenseite des medizinischen Geräts oder des Reinigungskorbs vorhandener Barcode und/oder ein QR-Code und/oder ein an oder in dem medizinischen Gerät oder dem Reinigungskorb vorhandener RFID-Tag ist.

Entsprechend handelt es sich bei dem berührungslos arbeitenden Sensor um einen Sensor zur Erfassung eines oder mehrerer der genannten Kennzeichnungsmerkmale. Beispielsweise ist der Sensor eine Kamera mit entsprechender Auswertelogik, welche zur Erfassung beispielsweise eines Barcodes oder eines QR-Codes eingerichtet ist. Sofern eine äußere Form des medizinischen Geräts und/oder des Reinigungskorbs als maschinenlesbares Kennzeichnungsmerkmal eingesetzt wird, ist der Sensor ferner an eine Datenbank angeschlossen, in welcher die verschiedenen äußeren Formen der medizinischen Geräte und/oder Reinigungskörbe hinterlegt sind. In vielen Fällen weisen medizinische Geräte und/oder Reinigungskörbe außerdem charakteristische Details auf, so dass eine Bilderfassung und anschließende Analyse auf diese charakteristischen Details gerichtet bzw. beschränkt werden kann. Es ist also mit anderen Worten ausreichend, diese charakteristischen Merkmale zur Identifikation zu erfassen.

Ferner ist insbesondere vorgesehen, dass der berührungslos arbeitende Sensor, sofern eine optische Erfassung realisiert ist, ein stereoskopisch arbeitendes System ist. Ausgehend von den mit einem stereoskopischen Kamerapaar erfassten stereoskopischen Bilddaten ist es möglich, eine Position, d. h. Ortskoordinaten, des erfassten medizinischen Geräts und/oder des Reinigungskorbs in einem Raum zu erfassen.

Der berührungslos arbeitende Sensor ist ferner insbesondere als RFID-Transponder, insbesondere als ein RFID-Interrogator, ausgebildet. So ist es möglich, den RFID-Tag an dem medizinischen Gerät oder dem Reinigungskorb auszulesen und beispielsweise über eine Laufzeitmessung dessen Entfernung von der Gerätefront zu bestimmen. Neben der reinen Bestimmung der Ortskoordinaten ist es ferner möglich, durch Auslesen des RFID-Tags den Reinigungskorb und/oder das medizinische Gerät oder einen Typ desselben auszulesen. Ausgehend von diesen Informationen wird das Aufbereitungsgerät in die Lage versetzt, beispielsweise Voreinstellungen zur Aufbereitung des medizinischen Geräts bereits vor Beginn des eigentlichen Reinigungsvorgangs vorzunehmen. Auch dies beschleunigt den Arbeitsablauf und erhöht den Durchsatz des Aufbereitungsgeräts.

Bei der Steuereinrichtung, welche mit dem berührungslos arbeitenden Sensor verbunden ist und eine Datenauswertung und -analyse vornimmt, handelt es sich beispielsweise um einen Computer, einen Mikrocontroller oder um eine Workstation.

Insbesondere ist der berührungslos arbeitende Sensor in die Gerätefront des Aufbereitungsgeräts integriert. Die Integration des berührungslos arbeitenden Sensors in die Gerätefront des Aufbereitungsgeräts stellt eine besonders kompakte Lösung dar.

Gemäß einer weiteren Ausführungsform ist vorgesehen, dass das Aufbereitungssystem dadurch fortgebildet ist, dass eine Mehrzahl von Aufbereitungsgeräten, eine zentrale Steuereinrichtung und eine Beladestation umfasst ist, wobei die zentrale Steuereinrichtung mit allen Aufbereitungsgeräten und der Beladestation über Datenverbindungen gekoppelt ist, und wobei die Beladestation zum Einlegen des medizinischen Geräts in den Reinigungskorb eingerichtet ist und einen weiteren berührungslos arbeitenden Sensor umfasst, der dazu eingerichtet ist, mit dem maschinenlesbaren Kennzeichnungsmerkmal des medizinischen Geräts und/oder des Reinigungskorbs zusammenzuwirken und ferner zu erkennen, ob sich das medizinische Gerät und/oder der Reinigungskorb an der Beladestation befinden und sofern dies festgestellt wird, ein Belegungssignal an die zentrale Steuereinrichtung des Aufbereitungssystems auszugeben, wobei die zentrale Steuereinrichtung als Reaktion auf den Empfang des Belegungssignals ein verfügbares Aufbereitungsgerät dem erkannten und an der Beladestation befindlichen medizinischen Gerät und/oder Reinigungskorb zuweist.

Unter einem verfügbaren Aufbereitungsgerät wird insbesondere ein Aufbereitungsgerät verstanden, welches sich zum Zeitpunkt der Erzeugung des Belegungssignals nicht in einem Betriebszustand befindet, in dem eine Reinigung oder Desinfektion von medizinischen Geräten in dem Aufbereitungsraum des entsprechenden Aufbereitungsgeräts durchgeführt wird. Mit anderen Worten ist ein verfügbares Aufbereitungsgerät dazu bereit, einen Reinigungskorb aufzunehmen und anschließend eine Reinigung und/oder Desinfektion des in dem Reinigungskorb vorhandenen medizinischen Geräts vorzunehmen. Der Aufbereitungsraum ist also insbesondere leer oder weist zumindest einen freien Platz zur Aufnahme des Reinigungskorbs auf. Es ist also insbesondere vorgesehen, dass zumindest ein Platz zur Aufnahme eines Reinigungskorbs in dem Aufbereitungsraum des Aufbereitungsgeräts noch verfügbar ist. Dies ist für Aufbereitungsgeräte relevant, welche dazu eingerichtet sind, eine Mehrzahl von Reinigungskörben gleichzeitig in ihrem Aufbereitungsraum aufzunehmen und eine parallele Reinigung und/oder Desinfektion von in den Aufbereitungskörben vorhandenen medizinischen Geräten vorzunehmen. Das Aufbereitungsgerät ist ferner insbesondere betriebsbereit.

Unter dem Zuweisen des Aufbereitungsgeräts zu dem in der Beladestation befindlichen medizinischen Gerät und/oder Reinigungskorb wird insbesondere ein Reservieren oder Blockieren bzw. ein eineindeutiges Zuweisen des medizinischen Geräts bzw. des Reinigungskorbs zu dem Aufbereitungsgerät bzw. zu zumindest einem Platz zur Aufnahme des Reinigungskorbs in dem Aufbereitungsraum des Aufbereitungsgeräts verstanden. Mit anderen Worten wird durch die Zuweisung verhindert, dass das Aufbereitungsgerät überbelegt wird, so dass der zugewiesene Reinigungskorb bzw. das zugewiesene medizinische Gerät in jedem Fall von dem zugewiesenen Aufbereitungsgerät aufgenommen wird, sobald die Vorbereitungen in der Beladestation abgeschlossen sind.

Vorteilhaft ist gemäß der genannten Ausführungsform sichergestellt, dass am Ende der in der Beladestation durchgeführten Vorbereitungsarbeiten in jedem Fall ein Aufbereitungsgerät zur Aufbereitung der medizinischen Geräte unmittelbar und ohne Wartezeit zur Verfügung steht. Dies trägt erheblich zur Verbesserung des Workflows in einem solchen Aufbereitungssystem bei. Die Effizienz des Aufbereitungssystems wird gesteigert.

Gemäß einer weiteren Ausführungsform ist das Aufbereitungssystem dadurch fortgebildet, dass der weitere Sensor ferner dazu eingerichtet ist zu erkennen, ob das medizinische Gerät und/oder der Reinigungskorb von der Beladestation entfernt werden und sofern dies festgestellt wird, ein weiteres Türöffnungssignal an die zentrale Steuereinrichtung auszugeben, welche dazu eingerichtet ist, dieses an das zugeordnete Aufbereitungsgerät weiterzuleiten, wobei die Steuereinrichtung des zugeordneten Aufbereitungsgeräts dazu eingerichtet ist in Reaktion auf den Empfang dieses weiteren Türöffnungssignals den Zugang zu dem Behandlungsraum durch Öffnen der Tür zu ermöglichen.

Vorteilhaft wird durch den weiteren berührungslos arbeitenden Sensor, welcher in die Beladestation integriert ist, die Möglichkeit geschaffen, ein weiteres Türöffnungssignal bereitzustellen. Dies verbessert die Zuverlässigkeit der Türöffnungsfunktion. Ferner ist es möglich, sofern sich die Beladestation außerhalb des Blickfelds oder der Reichweite des berührungslos arbeitenden Sensors des Aufbereitungsgeräts befindet, trotzdem eine die anschließende Beladung antizipierende Türöffnung vorzunehmen. Dies ist besonders bei großen Aufbereitungsräumen vorteilhaft.

Der weitere berührungslos arbeitende Sensor ist insbesondere in gleicher Weise ausgestaltet, wie der berührungslos arbeitende Sensor. Gleiches gilt für die vorteilhaften Weiterbildungen des berührungslos arbeitenden Sensors, die in gleicher Weise auch den weiteren berührungslos arbeitenden Sensor betreffen.

Gemäß einer weiteren Ausführungsform ist vorgesehen, dass das Aufbereitungsgerät eine erste Tür und eine zweite Tür umfasst und der Aufbereitungsraum durch die an einander gegenüberliegenden Gerätefronten des Aufbereitungsgeräts vorhandenen Türen an einander gegenüberliegenden Seiten geschlossen oder schließbar ist, wobei die Gerätefront eine erste Gerätefront ist, welche die erste Tür umfasst und eine unreine Seite des Aufbereitungsgeräts ist, wobei eine zweite Gerätefront, welche die zweite Tür umfasst, eine reine Seite des Aufbereitungsgeräts ist, und wobei die Steuereinrichtung des Aufbereitungsgeräts dazu eingerichtet ist, in Reaktion auf das empfangene Türöffnungssignal den Zugang zu dem Behandlungsraum von der unreinen Seite her durch Öffnen der ersten Tür zu ermöglichen.

Eine automatische Türöffnung, welche vorgenommen wird, sobald sich ein Reinigungskorb und/oder ein medizinisches Gerät dieser Tür nähern, ist für die unreine Seite eines Aufbereitungsgeräts besonders vorteilhaft. Aufbereitungsgeräte, deren Aufbereitungsraum durch zwei einander gegenüberliegende Türen geschlossen ist, sind typischerweise für hohe Durchsätze konzipiert, so dass die vorauseilende automatische Türöffnung für solche Geräte besonders vorteilhaft ist.

Unter einem "Ermöglichen", die Tür des Behandlungsraums zu öffnen, wird im Kontext der vorliegenden Erfindung insbesondere verstanden, dass die Steuereinrichtung des Aufbereitungsgeräts dazu eingerichtet ist, beispielsweise einen motorischen oder hydraulischen Antrieb dieser Tür derart anzusteuern, dass eine Türöffnung vorgenommen wird. Es ist ebenso möglich, dass die Steuereinrichtung einen Verriegelungsmechanismus entriegelt, so dass die Tür beispielsweise durch ein Gasdruck- oder Federelement selbsttätig aufschwingt. In einem solchen Fall ist die Freigabe der Verriegelungsvorrichtung die zum Öffnen der Tür erforderliche Maßnahme. Bevorzugt wird seitens der Steuereinrichtung ein elektrischer Antrieb angesteuert, welcher elektromotorisch ein Öffnen der Tür des Behandlungsraums bewirkt.

Die erfindungsgemäße Aufgabe wird ferner gelöst durch ein Verfahren zum Betreiben eines Aufbereitungssystems nach einem oder mehreren der genannten Ausführungsformen, wobei dieses Verfahren dadurch fortgebildet ist, dass der Sensor zeitabhängig Ortskoordinaten des medizinischen Geräts und/oder des Reinigungskorbs erfasst und an die Steuereinrichtung ausgibt, wobei die Steuereinrichtung anhand der zeitabhängigen Ortskoordinaten eine Bewegungsanalyse des medizinischen Geräts und/oder des Reinigungskorbs vornimmt und feststellt, ob sich das medizinische Gerät und/oder der Reinigungskorb auf die in der Gerätefront vorhandene Tür des Aufbereitungsgeräts zubewegen und sofern die Steuereinrichtung dies feststellt, ein Türöffnungssignal generiert und den Zugang zu dem Behandlungsraum durch Öffnen der Tür ermöglicht, wobei die Steuereinrichtung aus den zeitabhängigen Ortskoordinaten eine Raumkurve des medizinischen Geräts und/oder des Reinigungskorbs bestimmt und sofern die Raumkurve für zumindest eine vorgegebene Wegstrecke und/oder zumindest eine vorgegebene Zeitspanne und/oder mit zumindest einer vorgegebenen Geschwindigkeit in Richtung der in der Gerätefront vorhandenen Tür des Aufbereitungsgeräts verläuft, dies als Hinweis darauf wertet, dass sich das medizinische Gerät und/oder der Reinigungskorb auf die Tür des Aufbereitungsgeräts zubewegen.

Auf das Verfahren zum Betreiben des Aufbereitungssystems treffen gleiche oder ähnliche Vorteile zu, wie sie bereits im Hinblick auf das Aufbereitungssystem selbst erwähnt wurden, so dass diese nicht wiederholt werden sollen.

Das Verfahren ist ferner dadurch fortgebildet, dass die Steuereinrichtung aus den zeitabhängigen Ortskoordinaten eine Raumkurve des medizinischen Geräts und/oder des Reinigungskorbs bestimmt und sofern die Raumkurve für zumindest eine vorgegebene Wegstrecke und/oder zumindest eine vorgegebene Zeitspanne und/oder mit zumindest einer vorgegebenen Geschwindigkeit in Richtung der in der Gerätefront vorhandene Tür des Aufbereitungsgeräts verläuft, dies als Hinweis darauf wertet, dass sich das medizinische Gerät und/oder der Reinigungskorb auf die Tür des Aufbereitungsgeräts zubewegen.

Vorteilhaft ist das Verfahren ferner dadurch fortgebildet, dass die Steuereinrichtung ausgehend von den zeitabhängigen Ortskoordinaten feststellt, ob sich das medizinische Gerät und/oder der Reinigungskorb innerhalb eines an die Gerätefront angrenzenden Ladebereichs befinden und sofern dies der Fall ist, dies als Hinweis darauf wertet, dass sich das medizinische Gerät und/oder der Reinigungskorb auf die Tür des Aufbereitungsgeräts zubewegen.

Gemäß einer weiteren Ausführungsform ist das Verfahren vorteilhaft dadurch fortgebildet, dass das Aufbereitungssystem eine Mehrzahl von Aufbereitungsgeräten, eine zentrale Steuereinrichtung und eine Beladestation umfasst, wobei die zentrale Steuereinrichtung mit allen Aufbereitungsgeräten und der Beladestation über Datenverbindungen gekoppelt ist, wobei die Beladestation einen weiteren berührungslos arbeitenden Sensor umfasst, und wobei mit dem weiteren berührungslos arbeitenden Sensor erfasst wird, ob sich das medizinische Gerät und/oder der Reinigungskorb an der Beladestation befinden und sofern dies festgestellt wird, der weitere Sensor ein Belegungssignal an die zentrale Steuereinrichtung des Aufbereitungssystems ausgibt, wobei die zentrale Steuereinrichtung dieses Signal empfängt und als Reaktion auf den Empfang dieses Signals ein verfügbares Aufbereitungsgerät dem erkannten und an der Beladestation befindlichen medizinischen Gerät und/oder Reinigungskorb zuweist.

Insbesondere ist bei einem solchen Verfahren vorgesehen, dass der weitere Sensor ferner erkennt, ob das medizinische Gerät und/oder der Reinigungskorb von der Beladestation entfernt werden und sofern dies festgestellt wird, ein weiteres Türöffnungssignal an die zentrale Steuereinrichtung ausgibt, wobei die zentrale Steuereinrichtung dieses weitere Türöffnungssignal an das zugeordnete Aufbereitungsgerät weiterleitet, und wobei die Steuereinrichtung des zugeordneten Aufbereitungsgeräts in Reaktion auf den Empfang dieses weiteren Türöffnungssignals den Zugang zu dem Behandlungsraum durch Öffnen der Tür ermöglicht.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: schematisch einen medizinischen Arbeitsplatz, umfassend ein Aufbereitungssystem zum Reinigen und/oder Desinfizieren von medizinischen Geräten,
- Fig. 2: ein Aufbereitungsgerät in schematischer perspektivischer Ansicht.

In den Zeichnungen sind jeweils gleiche oder gleichartige Elemente und/oder Teile mit denselben Bezugsziffern versehen, so dass von einer erneuten Vorstellung jeweils abgesehen wird.

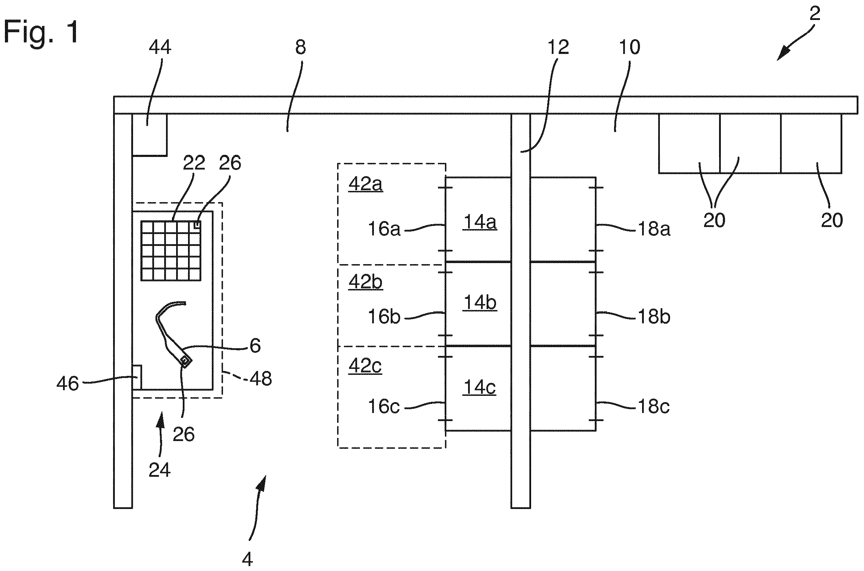

Fig. 1 zeigt in vereinfachter Draufsicht einen medizinischen Arbeitsplatz 2, in dem ein Aufbereitungssystem 4 zum Reinigen und/oder Desinfizieren von medizinischen Geräten 6 vorgesehen ist. Der medizinische Arbeitsplatz 2 umfasst einen Vorbereitungsraum 8 und einen Aufbewahrungsraum 10, die durch eine Trennwand 12 getrennt sind. In die Trennwand sind Aufbereitungsgeräte 14a, 14b, 14c integriert. Beispielhaft sind drei Aufbereitungsgeräte 14a, 14b, 14c dargestellt. Diese sind in Durchbrüchen der Trennwand 12 angeordnet und erstrecken sich beiderseits der Trennwand 12 in den Vorbereitungsraum 8 bzw. in den Aufbewahrungsraum 10.

Jedes der Aufbereitungsgeräte 14a, 14b, 14c umfasst eine erste Tür 16a, 16b, 16c, welche sich in einer ersten Gehäusefront des jeweiligen Aufbereitungsgeräts 14a, 14b, 14c befindet, die dem Vorbereitungsraum 8 zugewandt ist. Diese Seite der Aufbereitungsgeräte 14a, 14b, 14c ist die unreine Seite. Von dieser Seite werden die Aufbereitungsgeräte 14a, 14b, 14c beladen, d. h. zu reinigende medizinische Geräte 6 werden einem im Inneren der Aufbereitungsgeräte 14a, 14b, 14c liegenden und nicht dargestellten Aufbereitungsraum zugeführt. Dieser Aufbereitungsraum ist an der unreinen Seite von der ersten Tür 16a, 16b, 16c und an seiner gegenüberliegenden reinen Seite von einer zweiten Tür 18a, 18b, 18c begrenzt. Die zweiten Türen 18a, 18b, 18c der Aufbereitungsgeräte 14a, 14b, 14c liegen in einer zweiten Gehäusefront, welche dem Aufbewahrungsraum 10 zugewandt ist. Von dieser Seite werden den Aufbereitungsgeräten 14a, 14b, 14c die aufbereiteten, d.h. gereinigten und/oder desinfizierten medizinischen Geräte 6 entnommen und zur Aufbewahrung in entsprechende Aufbewahrungsschränke 20 verbracht. Dort lagern die medizinischen Geräte 6 bis zu ihrer Verwendung.

Das Aufbereitungssystem 4 zum Reinigen und/oder Desinfizieren von medizinischen Geräten 6 umfasst neben den Aufbereitungsgeräten 14a, 14b, 14c insbesondere zumindest ein medizinisches Gerät 6, beispielsweise ein Endoskop, und/oder einen Reinigungskorb 22.

In Fig. 1 sind der Reinigungskorb 22 und das medizinische Gerät 6 an einer Beladestation 24 dargestellt. Bei der Beladestation 24 handelt es sich beispielsweise um einen geeigneten Tisch oder eine Ablage. Die Beladestation 24 ist dazu eingerichtet, das medizinische Gerät 6 in den Reinigungskorb 22 einzulegen und außerdem insbesondere nicht dargestellte Adapter des Reinigungskorbs 22 an das medizinische Gerät 6 anzuschließen. Über diese Adapter wird während des Aufbereitungsvorgangs Spülflüssigkeit den inneren Kanälen des medizinischen Geräts 6 zugeführt, so dass diese gereinigt, desinfiziert bzw. gespült werden. Sobald die Beladung des Reinigungskorbs 22 abgeschlossen ist, wird dieser in eines der Aufbereitungsgeräte 14a, 14b, 14c geladen. Zu diesem Zweck trägt eine mit der Aufbereitung der medizinischen Geräte 6 betraute Person den beladenen Reinigungskorb 22 von der Beladestation 24 zu einem der Aufbereitungsgeräte 14a, 14b, 14c.

Das medizinische Gerät 6 und/oder der Reinigungskorb 22 sind mit einem maschinenlesbaren Kennzeichnungsmerkmal 26 versehen. Es ist gemäß verschiedenen Ausführungsbeispielen möglich, dass entweder das medizinische Gerät 6 oder der Reinigungskorb 22 mit einem maschinenlesbaren Kennzeichnungsmerkmal 26 versehen sind oder aber sowohl das medizinische Gerät 6 als auch der Reinigungskorb 22 jeweils mit einem maschinenlesbaren Kennzeichnungsmerkmal 26 versehen sind.

Bei dem maschinenlesbaren Kennzeichnungsmerkmal 26 handelt es sich beispielsweise um einen Barcode oder um einen QR-Code.

Ferner ist es möglich, ein RFID-Tag als maschinenlesbares Kennzeichnungsmerkmal 26 zu verwenden. Es ist ferner insbesondere vorgesehen, dass die äußere Form des medizinischen Geräts 6 und/oder die äußere Form des Reinigungskorbs 22 als Kennzeichnungsmerkmal 26 dienen. Es ist ferner bevorzugt möglich, dass lediglich ein Teil der äußeren Form des medizinischen Geräts 6 oder des Reinigungskorbs 22, beispielsweise ein besonders charakteristischer Teil der äußeren Form des jeweiligen Gegenstands, als maschinenlesbares Kennzeichnungsmerkmal 26 verwendet wird.

Das oder die maschinenlesbare(n) Kennzeichnungsmerkmal(e) 26 wirken mit einem berührungslos arbeitenden Sensor 28 zusammen, der insbesondere in eine dem Vorbereitungsraum 8 zugewandte Maschinenfront der Aufbereitungsgeräte 14a, 14b, 14c integriert ist.

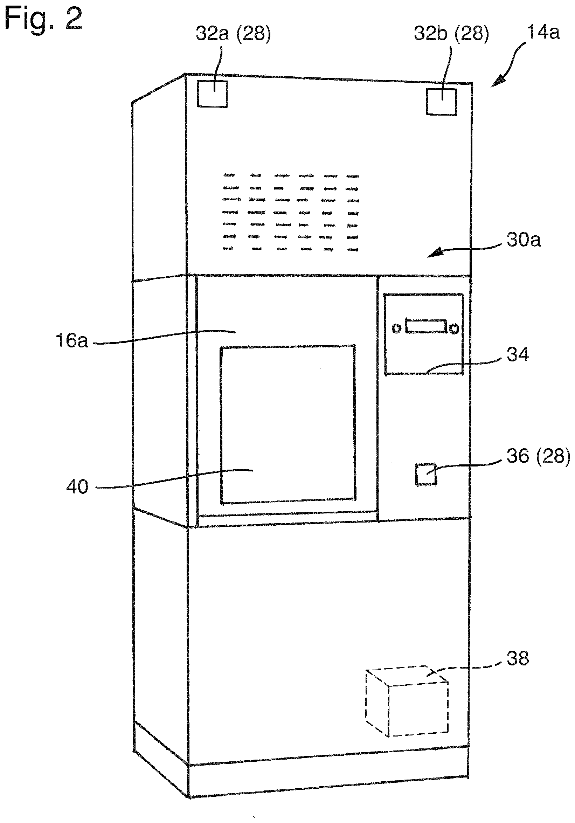

Fig. 2 zeigt in vereinfachter perspektivischer Ansicht beispielhaft das erste Aufbereitungsgerät 14a von seiner unreinen Seite her. In die erste Gehäusefront 30a, welche dem Vorbereitungsraum 8 zugewandt ist, sind im oberen Bereich eine erste und eine zweite Kamera 32a, 32b integriert, deren Blickrichtung in den Vorbereitungsraum 8 hineingerichtet ist. Gemäß einem weiteren Ausführungsbeispiel ist lediglich eine Kamera 32a, 32b vorgesehen. Ferner sind in die erste Gehäusefront 30a neben der ersten Tür 16a ein Bedienfeld 34 und ein RFID-Interrogator 36 integriert. Es ist ferner bevorzugt vorgesehen, dass der RFID-Interrogator 36 in das Bedienfeld 34 integriert ist. Bei der ersten und zweiten Kamera 32a, 32b und dem RFID-Interrogator 36 handelt es sich um berührungslos arbeitende Sensoren 28, die dazu eingerichtet sind, mit dem maschinenlesbaren Kennzeichnungsmerkmal 26 des medizinischen Geräts 6 und/oder des Reinigungskorbs 22 zusammenzuwirken.

Beispielsweise ist das Kamerapaar, bestehend aus der ersten und der zweiten Kamera 32a, 32b, dazu eingerichtet, einen Barcode oder einen QR-Code an dem medizinischen Gerät 6 und/oder an dem Reinigungskorb 22 zu erfassen. Alternativ oder zusätzlich ist vorgesehen, dass das Kamerapaar 32a, 32b eine äußere Form des medizinischen Geräts 6 und/oder des Reinigungskorbs 22 erfasst. Die beiden Kameras 32a, 32b bilden insbesondere ein stereoskopisches Kamerapaar zur Erfassung stereoskopischer Bildpaare. Insbesondere ist ebenso die Erfassung mit lediglich einer Kamera 32a, 32b vorgesehen. Der RFID-Interrogator 36 ist dazu eingerichtet, mit einem RFID-Tag zusammenzuwirken, welches sich als maschinenlesbares Kennzeichnungsmerkmal 26 an dem medizinischen Gerät 6 und/oder an dem Reinigungskorb 22 befindet. Das Aufbereitungsgerät 14a weist einen oder mehrere der berührungslos arbeitenden Sensoren 28 auf.

Die berührungslos arbeitenden Sensoren 28 sind dazu eingerichtet, zeitabhängige Ortskoordinaten des medizinischen Geräts 6 und/oder des Reinigungskorbs 22 zu erfassen. Das bedeutet, dass mit Hilfe der berührungslos arbeitenden Sensoren 28 die Position des medizinischen Geräts 6 und/oder des Reinigungskorbs 22 in dem Vorbereitungsraum 8 erfasst wird. Sofern es sich bei dem berührungslos arbeitenden Sensor 28 um ein stereoskopisches Kamerapaar 32a, 32b handelt, ist es anhand der stereoskopischen Bildpaare möglich, die Position des entsprechenden Gegenstands im Vorbereitungsraum 8 zu bestimmen. Sofern lediglich eine Kamera 32a, 32b eingesetzt wird, ist es möglich, die Position und den Abstand des medizinischen Geräts 8 und/oder des Reinigungskorbs 22 anhand von dessen Bildgröße und Relation zu den übrigen im Raum befindlichen Objekten zu bestimmen. Sofern RFID-Tags als maschinenlesbare Kennzeichnungsmerkmale 26 eingesetzt werden, ist es mit Hilfe des RFID-Interrogator 36 möglich, zumindest eine Entfernung des medizinischen Geräts 6 und/oder des Reinigungskorbs 22 vom Aufbereitungsgerät 14a zu bestimmen.

Zur Ermittlung der Ortskoordinaten sind die berührungslos arbeitenden Sensoren 28 mit einer Steuereinrichtung 38 verbunden. Die Steuereinrichtung 38 ist insbesondere ein Teil des Aufbereitungsgeräts 14a und beispielsweise als Computer oder Mikrocontroller ausgeführt. Sie ist dazu eingerichtet, ausgehend von zeitabhängigen Ortskoordinaten des medizinischen Geräts 6 und/oder des Reinigungskorbs 22 eine Bewegungsanalyse vorzunehmen. Die Steuereinrichtung 38 ist ferner dazu eingerichtet, festzustellen, ob sich das medizinische Gerät 6 und/oder der Reinigungskorb 22 auf die erste Gehäusefront 30a, genauer auf die in der ersten Gehäusefront 30a vorhandene erste Tür 16a, des Aufbereitungsgeräts 14a zubewegen. Sofern dies festgestellt wird, generiert die Steuereinrichtung 38 ein Türöffnungssignal, um den Zugang zu einem im Inneren des Aufbereitungsgeräts 14a vorhandenen Behandlungsraum freizugeben, der durch eine in die erste Tür 16a eingebaute Scheibe 40 sichtbar ist.

Insbesondere ist die Steuereinrichtung 38 dazu eingerichtet, aus den zeitabhängigen Ortskoordinaten des maschinenlesbaren Kennzeichnungsmerkmals 26 eine Raumkurve bzw. Trajektorie zu berechnen. Es wird berechnet, zu welchem Zeitpunkt sich das medizinische Gerät 6 und/oder der Reinigungskorb 22 an welchem Punkt im Vorbereitungsraum 8 befinden. Anhand dieser Informationen ist die Steuereinrichtung 38 in der Lage, Ort, Richtung und Geschwindigkeit des medizinischen Geräts 6 und/oder des Reinigungskorbs 22 zu bestimmen.

Weist die Raumkurve einen Abschnitt auf, der größer als eine vorgegebene Wegstrecke ist und in Richtung der ersten Gehäusefront 30a weist, wird dies als Hinweis darauf gewertet, dass das medizinische Gerät 6 und/oder der Reinigungskorb 22 auf die Tür 16a des Aufbereitungsgeräts 14a zubewegt werden. Dies gilt insbesondere, wenn der Abschnitt der Raumkurve länger als eine vorgegebene Wegstrecke ist, und es sich bei diesem Abschnitt um den aktuellsten, d.h. letzten Abschnitt der Raumkurve handelt.

Ferner ist insbesondere vorgesehen, dass, sofern die Raumkurve für zumindest eine vorgegebene Zeitspanne in Richtung der ersten Gehäusefront 30a des Aufbereitungsgeräts 14a weist, dies als Hinweis darauf gewertet wird, dass das medizinische Gerät 6 und/oder der Reinigungskorb 22 auf die Tür 16a des Aufbereitungsgeräts 14a zubewegt werden. Gleiches gilt, sofern die Raumkurve eine Geschwindigkeit anzeigt, die eine vorgegebene Geschwindigkeit des medizinischen Geräts 6 und/oder des Reinigungskorbs 22 überschreitet und der Geschwindigkeitsvektor in Richtung des Aufbereitungsgeräts 14a zeigt.

Alle Kriterien sind einzeln oder kumulativ einsetzbar, um festzustellen, dass sich das medizinische Gerät 6 und/oder der Reinigungskorb 22 auf die Tür 16a des Aufbereitungsgeräts 14a zubewegen. So wird festgestellt, dass die mit der Aufbereitung des medizinischen Geräts 6 betraute Person sich innerhalb des Vorbereitungsraums 8 von der Beladestation 24 auf den Weg zu einem der Aufbereitungsgeräte 14a, 14b, 14c macht. Die zuvor beispielhaft im Hinblick auf das erste Aufbereitungsgerät 14a erläuterten Aspekte treffen analog auf das zweite und dritte Aufbereitungsgerät 14b, 14c zu.

Ergreift eine mit der Aufbereitung des medizinischen Geräts 6 betraute Person den bestückten Reinigungskorb 22 und bewegt sich auf eines der Aufbereitungsgeräte 14a, 14b, 14c zu, so wird die Tür 16a, 16b, 16c des Aufbereitungsgeräts 14a, 14b, 14c geöffnet, bevor die Reinigungsperson überhaupt das Aufbereitungsgerät 14a, 14b, 14c erreicht hat. So wird wertvolle Arbeits- und Prozesszeit eingespart und die Auslastung der Aufbereitungsgeräte 14a, 14b, 14c wird optimiert.

Gemäß einem weiteren Ausführungsbeispiel ist vorgesehen, dass die zeitabhängig erfassten Ortskoordinaten des medizinischen Geräts 6 und/oder des Reinigungskorbs 22 derart ausgewertet werden, dass festgestellt wird, ob sich das medizinische Gerät 6 und/oder der Reinigungskorb 22 in einem der Ladebereiche 42a, 42b, 42c der Aufbereitungsgeräte 14a, 14b, 14c befinden. Sofern dies festgestellt wird, wird dies ebenfalls als Hinweis darauf gewertet, dass sich das medizinische Gerät 6 und/oder der Reinigungskorb 22 auf die Tür 16a, 16b, 16c des Aufbereitungsgeräts 14a, 14b, 14c zubewegen.

Es ist ferner insbesondere vorgesehen, dass lediglich der Reinigungskorb 22 mit einem maschinenlesbaren Kennzeichnungsmerkmal 26 versehen ist. So ist es möglich, sowohl medizinische Geräte 6 mit also auch ohne maschinenlesbares Kennzeichnungsmerkmal 26 in einem Aufbereitungssystem 4 zu verwenden.

Zur weiteren Verbesserung des Workflows in dem medizinischen Arbeitsplatz 2 umfasst das Aufbereitungssystem 4 ferner eine zentrale Steuereinrichtung 44 und einen weiteren berührungslos arbeitenden Sensor 46, welcher an der Beladestation 24 vorgesehen ist. Die Aufbereitungsgeräte 14a, 14b, 14c sind ferner über lediglich aus Gründen der Übersichtlichkeit nicht dargestellte Datenleitungen mit der zentralen Steuereinrichtung 44 verbunden. Ebenso ist der weitere berührungslos arbeitende Sensor 46 mit der zentralen Steuereinrichtung 44 mittels einer Datenleitung verbunden.

Bezüglich der Ausgestaltung des weiteren berührungslos arbeitenden Sensors 46 gilt das zu dem berührungslos arbeitenden Sensor 28 des Aufbereitungsgeräts 14a bereits Gesagte. Der Sensor 28 und der weitere Sensors 46 sind identisch oder verschieden ausgestaltet. Mit anderen Worten ist der weitere berührungslos arbeitende Sensor 46 beispielsweise eine Kamera oder ein stereoskopisches Kamerapaar zur Erfassung stereoskopischer Bilddaten. Ebenso kann es sich um einen RFID-Interrogator 36 handeln. Der weitere berührungslos arbeitende Sensor 46 ist dazu vorgesehen, mit den maschinenlesbaren Kennzeichnungsmerkmalen 26 des medizinischen Geräts 6 bzw. des Reinigungskorbs 22 zusammenzuwirken.

Der Reinigungskorb 22 wird an der Beladestation 24 mit dem zu reinigenden medizinischen Gerät 6 beladen. Während dieses Vorgangs befindet sich sowohl der Reinigungskorb 22 als auch das medizinische Gerät 6 an der Beladestation 24, d.h. innerhalb eines Umgebungsbereichs 48 der Beladestation 24. Der weitere Sensor 46 ist dazu eingerichtet festzustellen, ob sich das medizinische Gerät 6 und/oder der Reinigungskorb 22 an der Beladestation 24 befinden. Wird dies festgestellt, so wird dem medizinischen Gerät 6 und/oder dem Reinigungskorb 22 ein verfügbares Aufbereitungsgerät 14a, 14b, 14c zugewiesen. Dies bedeutet mit anderen Worten, dass eines der Aufbereitungsgeräte 14a, 14b, 14c für das medizinische Gerät 6 bzw. den Reinigungskorb 22 reserviert wird. Sind die Aufbereitungsgeräte 14a, 14b, 14c oder zumindest eines davon dazu vorgesehen, mehr als einen einzigen Reinigungskorb 22 aufzunehmen, so wird zumindest ein Platz in einem dieser Aufbereitungsgeräte 14a, 14b, 14c für den Reinigungskorb 22 reserviert.

Ein Aufbereitungsgerät 14a, 14b, 14c wird dann als verfügbar angesehen, wenn dieses sich zum Zeitpunkt der Zuweisung nicht in einem Reinigungs- und/oder Desinfektionsvorgang befindet, sondern betriebsbereit zur Verfügung steht, einen solchen unmittelbar zu beginnen. Um eine solche Zuweisung zwischen dem medizinischen Gerät 6 und/oder dem Reinigungskorb 22 und einem verfügbaren Aufbereitungsgerät 14a, 14b, 14c vorzunehmen, wird ein Belegungssignal in dem weiteren berührungslos arbeitenden Sensor 46 generiert und an die zentrale Steuereinrichtung 44 übertragen. Diese hält Informationen betreffend den Betriebszustand der Aufbereitungsgeräte 14a, 14b, 14c vor und ist in der Lage, eine Zuordnung zwischen dem medizinischen Gerät 6 und/oder dem Reinigungskorb 22 und einem verfügbaren Aufbereitungsgerät 14a, 14b, 14c zu treffen. Das entsprechende Aufbereitungsgerät 14a, 14b, 14c ist anschließend reserviert, so dass nach Beendigung der Vorbereitungsarbeiten für den Reinigungskorb 22 in jedem Fall ein Einschub in einem der Aufbereitungsgeräte 14a, 14b, 14c zur Verfügung steht, so dass nach Einschieben des Reinigungskorbs 22 der Aufbereitungsvorgang unmittelbar beginnt.

Es ist ferner insbesondere vorgesehen, dass zur Verbesserung des Arbeitsablaufs in dem medizinischen Arbeitsplatz 2 der weitere berührungslos arbeitende Sensor 46 dazu eingerichtet ist zu erkennen, wann und ob das medizinische Gerät 6 und/oder der Reinigungskorb 22 die Beladestation 24 verlassen. Dies wird beispielsweise festgestellt, indem eine Ortskoordinate des medizinischen Geräts 6 bzw. des Reinigungskorbs 22 bestimmt wird und überprüft wird, ob diese Ortskoordinate sich innerhalb des Umgebungsbereichs 48 der Beladestation 24 befindet. Verlässt der Reinigungskorb 22 einschließlich des medizinischen Geräts 6 den Umgebungsbereich 48 der Beladestation 24, so wird dies als Hinweis darauf gewertet, dass sich der Reinigungskorb 22 auf das zugeordnete Aufbereitungsgerät 14a, 14b, 14c zubewegt. Es wird ein Türöffnungssignal von der Steuereinrichtung 38 des Aufbereitungsgeräts 14a (vgl. Fig. 2) generiert, so dass die Tür 16a bereits geöffnet ist, bevor die mit der Aufbereitung der medizinischen Geräte 6 betraute Person, welche den Reinigungskorb 22 zuvor der Beladestation 24 entnommen hat, an dem Aufbereitungsgerät 14a ankommt. Somit ist vorteilhaft sichergestellt, dass der Arbeitsablauf in dem medizinischen Arbeitsplatz 2 effektiv vonstattengeht und die Aufbereitungsgeräte 14a, 14b, 14c mit hoher Effizienz betrieben werden.

Bei einem Verfahren zum Betreiben eines Aufbereitungssystems 4, wie es in den Fig. 1 und 2 gezeigt ist, werden von einem berührungslos arbeitenden Sensor 28 zeitabhängig Ortskoordinaten des medizinischen Geräts 6 und/oder des Reinigungskorbs 22 erfasst. Diese werden an eine Steuereinrichtung 38 des Aufbereitungsgeräts 14a ausgegeben. Die Steuereinrichtung 38 nimmt anhand dieser zeitabhängigen Ortskoordinaten eine Bewegungsanalyse des medizinischen Geräts 6 und/oder des Reinigungskorbs 22 vor. Sie stellt ferner fest, ob sich das medizinische Gerät 6 und/oder der Reinigungskorb 22 auf die erste Gehäusefront 30a, genauer auf die erste Tür 16a innerhalb der ersten Gehäusefront 30a des Aufbereitungsgeräts 14a, zubewegen. Wird dies festgestellt, erzeugt die Steuereinrichtung 38 ein Türöffnungssignal, so dass der Zugang zu dem Behandlungsraum durch Öffnen der Tür 16a möglich ist.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 2: medizinischer Arbeitsplatz
- 4: Aufbereitungssystem
- 6: medizinisches Gerät
- 8: Vorbereitungsraum
- 10: Aufbewahrungsraum
- 12: Trennwand
- 14a, 14b, 14c: Aufbereitungsgerät
- 16a, 16b, 16c: erste Tür
- 18a, 18b, 18c: zweite Tür
- 20: Aufbewahrungsschrank
- 22: Reinigungskorb
- 24: Beladestation
- 26: maschinenlesbares Kennzeichnungsmerkmal
- 28: berührungslos arbeitender Sensor
- 30a: erste Gehäusefront
- 32a: erste Kamera
- 32b: zweite Kamera
- 34: Bedienfeld
- 36: RFID-Interrogator
- 38: Steuereinrichtung
- 40: Scheibe
- 42a, 42b, 42c: Ladebereich
- 44: zentrale Steuereinrichtung
- 46: weiterer berührungslos arbeitender Sensor
- 48: Umgebungsbereich

## Patentansprüche

1. Aufbereitungssystem (4) zum Reinigen und/oder Desinfizieren von medizinischen Geräten (6), insbesondere Endoskopen, umfassend zumindest ein Aufbereitungsgerät (14a, 14b, 14c), wobei ein Aufbereitungsraum des Aufbereitungsgeräts (14a, 14b, 14c) dazu vorgesehen ist, dass zumindest ein, insbesondere in dem Reinigungskorb (22) angeordnetes medizinisches Gerät (6) in einem Reinigungs- und/oder Desinfiziervorgang innerhalb des Aufbereitungsraums gereinigt und/oder desinfiziert wird, und wobei der Aufbereitungsraum durch eine an einer Gerätefront (30a) des Aufbereitungsgeräts (14a, 14b, 14c) vorhandene Tür (16a, 16b, 16c) geschlossen oder schließbar ist, **dadurch gekennzeichnet, dass** das medizinische Gerät (6) und/oder der Reinigungskorb (22) ein maschinenlesbares Kennzeichnungsmerkmal (26) umfassen und das Aufbereitungsgerät (14a, 14b, 14c) einen berührungslos arbeitenden Sensor (28) umfasst, der dazu eingerichtet ist mit dem maschinenlesbaren Kennzeichnungsmerkmal (26) zusammenzuwirken, wobei der Sensor (28) ferner dazu eingerichtet ist zeitabhängig Ortskoordinaten des medizinischen Geräts (6) und/oder des Reinigungskorbs (22) zu erfassen und an eine Steuereinrichtung (38) des Aufbereitungsgeräts (14a, 14b, 14c) auszugeben, wobei die Steuereinrichtung (38) dazu eingerichtet ist, ausgehend von den zeitabhängigen Ortskoordinaten, eine Bewegungsanalyse des erfassten medizinischen Geräts (6) und/oder Reinigungskorbs (22) vorzunehmen und festzustellen, ob sich das medizinischen Gerät (6) und/oder der Reinigungskorb (22) auf die in der Gerätefront (30a) vorhandene Tür (16a, 16b, 16c) des Aufbereitungsgeräts (14a, 14b, 14c) zubewegen und sofern dies festgestellt wird, ein Türöffnungssignal zu generieren und den Zugang zu dem Behandlungsraum durch Öffnen der Tür (16a, 16b, 16c) zu ermöglichen, wobei die Steuereinrichtung (38) dazu eingerichtet ist, aus den zeitabhängigen Ortskoordinaten eine Raumkurve des medizinischen Geräts (6) und/oder des Reinigungskorbs (22) zu bestimmen und sofern diese Raumkurve für zumindest eine vorgegebene Wegstrecke und/oder zumindest eine vorgegebene Zeitspanne und/oder mit zumindest einer vorgegebenen Geschwindigkeit in Richtung der in der Gerätefront (30a) des Aufbereitungsgeräts (14a, 14b, 14c) vorhandenen Tür (16a, 16b, 16c) verläuft, dies als Hinweis darauf zu werten, dass sich das medizinische Gerät (6) und/oder der Reinigungskorb (22) auf die Tür (16a, 16b, 16c) des Aufbereitungsgeräts (14a, 14b, 14c) zubewegen.

2. Aufbereitungssystem (4) nach Anspruch 1, **dadurch gekennzeichnet, dass** die Steuereinrichtung (38) dazu eingerichtet ist, anhand der zeitabhängigen Ortskoordinaten festzustellen, ob sich das medizinische Gerät (6) und/oder der Reinigungskorb (22) innerhalb eines Ladebereichs (42a, 42b, 42c) befinden, der an die die Tür (16a, 16b, 16c) umfassende Gerätefront (30a) angrenzt, und sofern dies der Fall ist, dies als Hinweis darauf zu werten, dass sich das medizinische Gerät (6) und/oder der Reinigungskorb (22) auf die Tür (16a, 16b, 16c) des Aufbereitungsgeräts (14a, 14b, 14c) zubewegen.

3. Aufbereitungssystem (4) nach Anspruch 1 oder 2, **dadurch gekennzeichnet, dass** ausschließlich der Reinigungskorb (22) zur Aufnahme des zumindest einen medizinischen Geräts (6) mit dem maschinenlesbaren Kennzeichnungsmerkmal (26) versehen ist.

4. Aufbereitungssystem (4) nach einem der Ansprüche 1 bis 3, **dadurch gekennzeichnet, dass** das maschinenlesbare Kennzeichnungsmerkmal (26) eine äußere Form des medizinischen Geräts (6) und/oder des Reinigungskorbs (22) ist und/oder das maschinenlesbare Kennzeichnungsmerkmal (26) ein auf einer Außenseite des medizinischen Geräts (6) oder des Reinigungskorbs (22) vorhandener Barcode und/oder ein QR-Code und/oder ein an oder in dem medizinischen Gerät (6) oder dem Reinigungskorb (22) vorhandener RFID-Tag ist.

5. Aufbereitungssystem (4) nach einem der Ansprüche 1 bis 4, **dadurch gekennzeichnet, dass** eine Mehrzahl von Aufbereitungsgeräten (14a, 14b, 14c), eine zentrale Steuereinrichtung (44) und eine Beladestation (24) umfasst ist, wobei die zentrale Steuereinrichtung (44) mit allen Aufbereitungsgeräten (14a, 14b, 14c) und der Beladestation (24) über Datenverbindungen gekoppelt ist, und wobei die Beladestation (24) zum Einlegen des medizinischen Geräts (6) in den Reinigungskorb (22) eingerichtet ist und einen weiteren berührungslos arbeitenden Sensor (46) umfasst, der dazu eingerichtet ist mit dem maschinenlesbaren Kennzeichnungsmerkmal (26) des medizinischen Geräts (6) und/oder des Reinigungskorbs (22) zusammenzuwirken und ferner zu erkennen, ob sich das medizinische Gerät (6) und/oder der Reinigungskorb (22) an der Beladestation (24) befinden und sofern dies festgestellt wird, ein Belegungssignal an die zentrale Steuereinrichtung (44) des Aufbereitungssystems (4) auszugeben, wobei die zentrale Steuereinrichtung (44) als Reaktion auf den Empfang des Belegungssignals ein verfügbares Aufbereitungsgerät (14a, 14b, 14c) dem erkannten und an der Beladestation (24) befindlichen medizinischen Gerät (6) und/oder Reinigungskorb (22) zuweist.

6. Aufbereitungssystem (4) nach Anspruch 5, **dadurch gekennzeichnet, dass** der weitere Sensor (46) ferner dazu eingerichtet ist zu erkennen, ob das medizinische Gerät (6) und/oder der Reinigungskorb (22) von der Beladestation (24) entfernt werden und sofern dies festgestellt wird, ein weiteres Türöffnungssignal an die zentrale Steuereinrichtung (44) auszugeben, welche dazu eingerichtet ist, dieses an das zugeordnete Aufbereitungsgerät (14a, 14b, 14c) weiterzuleiten, wobei die Steuereinrichtung (38) des zugeordneten Aufbereitungsgeräts (14a, 14b, 14c) dazu eingerichtet ist in Reaktion auf den Empfang dieses weiteren Türöffnungssignals den Zugang zu dem Behandlungsraum durch Öffnen der Tür (16a, 16b, 16c) zu ermöglichen.

7. Aufbereitungssystem (4) nach einem der Ansprüche 1 bis 6, **dadurch gekennzeichnet, dass** das Aufbereitungsgerät (14a, 14b, 14c) eine erste Tür (16a, 16b, 16c) und eine zweite Tür (18a, 18b, 18c) umfasst und der Aufbereitungsraum durch die an einander gegenüberliegenden Gerätefronten des Aufbereitungsgeräts (14a, 14b, 14c) vorhandenen Türen (16a, 16b, 16c, 18a, 18b, 18c) an einander gegenüberliegenden Seiten geschlossen oder schließbar ist, wobei die Gerätefront eine erste Gerätefront (30a) ist, welche die erste Tür (16a, 16b, 16c) umfasst und eine unreine Seite des Aufbereitungsgeräts (14a, 14b, 14c) ist, wobei eine zweite Gerätefront, welche die zweite Tür (18a, 18b, 18c) umfasst, eine reine Seite des Aufbereitungsgeräts (14a, 14b, 14c) ist, und wobei die Steuereinrichtung (38) des Aufbereitungsgeräts (14a, 14b, 14c) dazu eingerichtet ist, in Reaktion auf das empfangene Türöffnungssignal den Zugang zu dem Behandlungsraum von der unreinen Seite her durch Öffnen der ersten Tür (16a, 16b, 16c) zu ermöglichen.

8. Verfahren zum Betreiben eines Aufbereitungssystems (4) nach einem der Ansprüche 1 bis 7, **dadurch gekennzeichnet, dass** der Sensor (28) zeitabhängig Ortskoordinaten des medizinischen Geräts (6) und/oder des Reinigungskorbs (22) erfasst und an die Steuereinrichtung (38) ausgibt, wobei die Steuereinrichtung (38) anhand der zeitabhängigen Ortskoordinaten eine Bewegungsanalyse des medizinischen Geräts (6) und/oder des Reinigungskorbs (22) vornimmt und feststellt, ob sich das medizinische Gerät (6) und/oder der Reinigungskorb (22) auf die in der Gerätefront (30a) vorhandene Tür (16a, 16b, 16c) des Aufbereitungsgeräts (14a, 14b, 14c) zubewegen und sofern die Steuereinrichtung (38) dies feststellt, ein Türöffnungssignal generiert und den Zugang zu dem Behandlungsraum durch Öffnen der Tür (16a, 16b, 16c) ermöglicht, wobei die Steuereinrichtung (38) aus den zeitabhängigen Ortskoordinaten eine Raumkurve des medizinischen Geräts (6) und/oder des Reinigungskorbs (22) bestimmt und sofern die Raumkurve für zumindest eine vorgegebene Wegstrecke und/oder zumindest eine vorgegebene Zeitspanne und/oder mit zumindest einer vorgegebenen Geschwindigkeit in Richtung der in der Gerätefront vorhandene Tür (16a, 16b, 16c) des Aufbereitungsgeräts (14a, 14b, 14c) verläuft, dies als Hinweis darauf wertet, dass sich das medizinische Gerät (6) und/oder der Reinigungskorb (22) auf die Tür (16a, 16b, 16c) des Aufbereitungsgeräts (14a, 14b, 14c) zubewegen.

9. Verfahren nach Anspruch 8, **dadurch gekennzeichnet, dass** die Steuereinrichtung (38) ausgehend von den zeitabhängigen Ortskoordinaten feststellt, ob sich das medizinische Gerät (6) und/oder der Reinigungskorb (22) innerhalb eines an die Gerätefront (30a) angrenzenden Ladebereichs (42a, 42b, 42c) befinden und sofern dies der Fall ist, dies als Hinweis darauf wertet, dass sich das medizinische Gerät (6) und/oder der Reinigungskorb (22) auf die Tür (16a, 16b, 16c) des Aufbereitungsgeräts (14a, 14b, 14c) zubewegen.

10. Verfahren nach einem der Ansprüche 8 bis 9, **dadurch gekennzeichnet, dass** das Aufbereitungssystem (4) eine Mehrzahl von Aufbereitungsgeräten (14a, 14b, 14c), eine zentrale Steuereinrichtung (44) und eine Beladestation (24) umfasst, wobei die zentrale Steuereinrichtung (44) mit allen Aufbereitungsgeräten (14a, 14b, 14c) und der Beladestation (24) über Datenverbindungen gekoppelt ist, wobei die Beladestation (24) einen weiteren berührungslos arbeitenden Sensor (46) umfasst, und wobei mit dem weiteren berührungslos arbeitenden Sensor (46) erfasst wird, ob sich das medizinische Gerät (6) und/oder der Reinigungskorb (22) an der Beladestation (24) befinden und sofern dies festgestellt wird, der weitere Sensor (46) ein Belegungssignal an die zentrale Steuereinrichtung (44) des Aufbereitungssystems (4) ausgibt, wobei die zentrale Steuereinrichtung (44) dieses Signal empfängt und als Reaktion auf dem Empfang dieses Signals ein verfügbares Aufbereitungsgerät (14a, 14b, 14c) dem erkannten und an der Beladestation (24) befindlichen medizinischen Gerät (6) und/oder Reinigungskorb (22) zuweist.

11. Verfahren nach Anspruch 10, **dadurch gekennzeichnet, dass** der weitere Sensor (46) ferner erkennt, ob das medizinische Gerät (6) und/oder der Reinigungskorb (22) von der Beladestation (24) entfernt werden und sofern dies festgestellt wird, ein weiteres Türöffnungssignal an die zentrale Steuereinrichtung (44) ausgibt, wobei die zentrale Steuereinrichtung (44) dieses weitere Türöffnungssignal an das zugeordnete Aufbereitungsgerät (14a, 14b, 14c) weiterleitet, und wobei die Steuereinrichtung (38) des zugeordneten Aufbereitungsgeräts (14a, 14b, 14c) in Reaktion auf den Empfang dieses weiteren Türöffnungssignals den Zugang zu dem Behandlungsraum durch Öffnen der Tür (16a, 16b, 16c) ermöglicht.

## Claims

1. A reprocessing system (4) for cleaning and/or disinfecting medical devices (6), in particular endoscopes, comprising at least one reprocessing device (14a, 14b, 14c), wherein a reprocessing chamber of the reprocessing device (14a, 14b, 14c) is provided for cleaning and/or disinfecting at least one medical device (6), in particular arranged in the cleaning basket (22), in a cleaning and/or disinfection process inside the reprocessing chamber, and wherein the reprocessing chamber is closed or can be closed by means of a door (16a, 16b, 16c) provided on a front panel (30a) of the reprocessing device (14a, 14b, 14c), **characterized in that** the medical device (6) and/or the cleaning basket (22) comprise a machine-readable identifying feature (26) and the reprocessing device (14a, 14b, 14c) comprises a contact-free operating sensor (28) which is configured to interact with the machine-readable identifying feature (26), wherein the sensor (28) is further configured to record location coordinates of the medical device (6) and/or the cleaning basket (22) in a time-dependent manner and to output said coordinates to a control unit (38) of the reprocessing device (14a, 14b, 14c), wherein the control unit (38) is configured, proceeding from the time-dependent location coordinates, to perform a movement analysis of the recorded medical device (6) and/or cleaning basket (22) and to determine whether the medical device (6) and/or the cleaning basket (22) are moving towards the door (16a, 16b, 16c) of the reprocessing device (14a, 14b, 14c) provided in the front panel (30a) and, if this is determined, to generate a door-opening signal and to allow access to the treatment chamber by opening the door (16a, 16b, 16c), wherein the control unit (38) is configured to determine a space curve of the medical device (6) and/or cleaning basket (22) from the time-dependent location coordinates and, if this space curve extends for at least a predefined distance and/or at least a predefined time period and/or at at least a predefined speed in the direction of the door (16a, 16b, 16c) provided in the front panel (30a) of the reprocessing device (14a, 14b, 14c), to take this as an indication that the medical device (6) and/or the cleaning basket (22) are moving towards the door (16a, 16b, 16c) of the reprocessing device (14a, 14b, 14c).

2. The reprocessing system (4) according to claim 1, **characterized in that** the control unit (38) is configured, based on the time-dependent location coordinates, to determine whether the medical device (6) and/or the cleaning basket (22) are located inside a loading region (42a, 42b, 42c) which adjoins the front panel (30a) comprising the door (16a, 16b, 16c) and, if this is the case, to take this as an indication that the medical device (6) and/or the cleaning basket (22) are moving towards the door (16a, 16b, 16c) of the reprocessing device (14a, 14b, 14c).

3. The reprocessing system (4) according to claim 1 or 2, **characterized in that** exclusively the cleaning basket (22) for receiving the at least one medical device (6) is equipped with the machine-readable identifying feature (26).

4. The reprocessing system (4) according to any one of claims 1 to 3, **characterized in that** the machine-readable identifying feature (26) is an outer shape of the medical device (6) and/or cleaning basket (2) and/or the machine-readable identifying feature (26) is a bar code provided on an outer face of the medical device (6) or cleaning basket (22) and/or a QR code and/or an RFID tag provided on or in the medical device (6) or cleaning basket (22).

5. The reprocessing system (4) according to any one of claims 1 to 4, **characterized in that** a plurality of reprocessing devices (14a, 14b, 14c), a central control unit (44), and a loading station (24) are comprised, wherein the central control unit (44) is coupled to all reprocessing devices (14a, 14b, 14c) and to the loading station (24) via data links, and wherein the loading station (24) is configured to place the medical device (6) in the cleaning basket (22) and comprises an additional contact-free operating sensor (46) which is configured to interact with the machine-readable identifying feature (26) of the medical device (6) and/or cleaning basket (22) and further to detect whether the medical device (6) and/or the cleaning basket (22) are located at the loading station (24) and, if this is determined, to output an occupancy signal to the central control unit (44) of the reprocessing system (4), wherein the central control unit (44) assigns an available reprocessing device (14a, 14b, 14c) to the detected medical device (6) and/or cleaning basket (22) located at the loading station (24) in response to receiving the occupancy signal.

6. The reprocessing system (4) according to claim 5, **characterized in that** the additional sensor (46) is further configured to detect whether the medical device (6) and/or the cleaning basket (22) are being removed from the loading station (24) and, if this is determined, to output an additional door-opening signal to the central control unit (44), which is configured to forward said signal to the allocated reprocessing device (14a, 14b, 14c), wherein the control unit (38) of the allocated reprocessing device (14a, 14b, 14c) is configured to allow access to the treatment chamber by opening the door (16a, 16b, 16c) in response to receiving said additional door-opening signal.

7. The reprocessing system (4) according to any one of claims 1 to 6, **characterized in that** the reprocessing device (14a, 14b, 14c) comprises a first door (16a, 16b, 16c) and a second door (18a, 18b, 18c) and the reprocessing chamber is closed or can be closed on opposing sides by means of the doors (16a, 16b, 16c, 18a, 18b, 18c) provided on opposing front panels of the reprocessing device (14a, 14b, 14c), wherein the front panel is a first front panel (30a) which comprises the first door (16a, 16b, 16c) and is an unclean side of the reprocessing device (14a, 14b, 14c), wherein a second front panel which comprises the second door (18a, 18b, 18c) is a clean side of the reprocessing device (14a, 14b, 14c), and wherein the control unit (38) of the reprocessing device (14a, 14b, 14c) is configured to allow access to the treatment chamber from the unclean side by opening the first door (16a, 16b, 16c) in response to the received door-opening signal.

8. A method for operating a reprocessing system (4) according to any one of claims 1 to 7, **characterized in that** the sensor (28) records location coordinates of the medical device (6) and/or cleaning basket (22) in a time-dependent manner and outputs said coordinates to the control unit (38), wherein the control unit (38) performs a movement analysis of the medical device (6) and/or cleaning basket (22) based on the time-dependent location coordinates and establishes whether the medical device (6) and/or the cleaning basket (22) are moving towards the door (16a, 16b, 16c) of the reprocessing device (14a, 14b, 14c) provided in the front panel (30a) and, if the control unit (38) establishes this, generates a door-opening signal and allows access to the treatment chamber by opening the door (16a, 16b, 16c), wherein the control unit (38) determines a space curve of the medical device (6) and/or cleaning basket (22) from the time-dependent location coordinates and, if the space curve extends for at least a predefined distance and/or at least a predefined time period and/or at at least a predefined speed in the direction of the door (16a, 16b, 16c) of the reprocessing device (14a, 14b, 14c) provided in the front panel, takes this as an indication that the medical device (6) and/or the cleaning basket (22) are moving towards the door (16a, 16b, 16c) of the reprocessing device (14a, 14b, 14c).

9. The method according to claim 8, **characterized in that** the control unit (38), proceeding from the time-dependent location coordinates, establishes whether the medical device (6) and/or the cleaning basket (22) are located inside a loading region (42a, 42b, 42c) adjoining the front panel (30a) and, if this is the case, takes this as an indication that the medical device (6) and/or the cleaning basket (22) are moving towards the door (16a, 16b, 16c) of the reprocessing device (14a, 14b, 14c).

10. The method according to any one of claims 8 to 9, **characterized in that** the reprocessing system (4) comprises a plurality of reprocessing devices (14a, 14b, 14c), a central control unit (44), and a loading station (24), wherein the central control unit (44) is coupled to all reprocessing devices (14a, 14b, 14c) and to the loading station (24) via data links, wherein the loading station (24) comprises an additional contact-free operating sensor (46), and wherein it is detected by means of the additional contact-free operating sensor (46) whether the medical device (6) and/or the cleaning basket (22) are located at the loading station (24) and, if this is determined, the additional sensor (46) outputs an occupancy signal to the central control unit (44) of the reprocessing system (4), wherein the central control unit (44) receives said signal and assigns an available reprocessing device (14a, 14b, 14c) to the detected medical device (6) and/or cleaning basket (22) located at the loading station (24) in response to receiving said signal.

11. The method according to claim 10, **characterized in that** the additional sensor (46) further detects whether the medical device (6) and/or the cleaning basket (22) are removed from the loading station (24) and, if this is determined, outputs an additional door-opening signal to the central control unit (44), wherein the central control unit (44) forwards said additional door-opening signal to the allocated reprocessing device (14a, 14b, 14c), and wherein the control unit (38) of the allocated reprocessing device (14a, 14b, 14c) allows access to the treatment chamber by opening the door (16a, 16b, 16c) in response to receiving said additional door-opening signal.

## Revendications

1. Système de traitement (4) pour le nettoyage et/ou la désinfection d'appareils médicaux (6), en particulier d'endoscopes, comprenant au moins un appareil de traitement (14a, 14b, 14c), un espace de traitement de l'appareil de traitement (14a, 14b, 14c) étant prévu pour qu'au moins un appareil médical (6), en particulier disposé dans le panier de nettoyage (22), soit nettoyé et/ou désinfecté, au cours d'un processus de nettoyage et/ou de désinfection à l'intérieur de l'espace de traitement, et l'espace de traitement étant fermé ou apte à être fermé par une porte (16a, 16b, 14c) présente sur une façade (30a) de l'appareil de préparation (14a, 14b, 14c), **caractérisé en ce que** l'appareil médical (6) et/ou le panier de nettoyage (22) comprennent un identifiant (26) lisible par machine, et l'appareil de traitement (14a, 14b, 14c) comprend un capteur (28) fonctionnant sans contact, qui est conçu pour coopérer avec l'identifiant (26) lisible par machine, le capteur (28) étant en outre conçu pour détecter, en fonction du temps, des coordonnées de lieu de l'appareil médical (6) et/ou du panier de nettoyage (22) et pour les délivrer à un dispositif de commande (38) de l'appareil de traitement (14a, 14b, 14c), le dispositif de commande (38) étant conçu pour effectuer, à partir des coordonnées de lieu dépendant du temps, une analyse de mouvement de l'appareil médical (6) et/ou du panier de nettoyage (22) saisi(e) et pour déterminer si l'appareil médical (6) et/ou le panier de nettoyage (22) se déplacent vers la porte (16a, 16b, 16c) située sur la façade (30a) de l'appareil de traitement (14a, 14b, 14c) et, si cela est détecté, pour générer un signal d'ouverture de porte et permettre l'accès à la salle de traitement par l'ouverture de la porte (16a, 16b, 16c), le dispositif de commande (38) étant conçu pour déterminer, à partir des coordonnées de lieu dépendant du temps, une courbe spatiale de l'appareil médical (6) et/ou du panier de nettoyage (22) et, si cette courbe spatiale s'étend sur au moins une distance prédéfinie et/ou au moins un laps de temps prédéfini et/ou avec au moins une vitesse prédéfinie en direction de la porte située sur la façade (30a) de l'appareil de traitement (14a, 14b, 14c), l'évalue comme étant une indication que l'appareil médical (6) et/ou le panier de nettoyage (22) se déplacent vers la porte (16a, 16b, 16c) de l'appareil de traitement (14a, 14b, 14c).

2. Système de traitement (4) selon la revendication 1, **caractérisé en ce que** le dispositif de commande (38) est agencé pour déterminer, en utilisant les coordonnées de lieu dépendant du temps, si l'appareil médical (6) et/ou le panier de nettoyage (22) se trouvent à l'intérieur d'une zone de chargement (42a, 42b, 42c), qui est adjacente à la porte (16a, 16b, 16c) comprenant la façade de l'appareil (30a), et, si c'est le cas, pour considérer cela comme une indication que l'appareil médical (6) et/ou le panier de nettoyage (22) se déplacent vers la porte (16a, 16b, 16c) de l'appareil de traitement (14a, 14b, 14c).

3. Système de traitement (4) selon la revendication 1 ou la revendication 2, **caractérisé en ce que** seul le panier de nettoyage (22) destiné à recevoir ledit au moins un appareil médical (6) est pourvu de l'identifiant (26) lisible par une machine.

4. Système de traitement (4) selon l'une des revendications 1 à 3, **caractérisé en ce que** l'identifiant lisible par machine (26) est une forme extérieure du dispositif médical (6) et/ou du panier de nettoyage (22), et/ou l'identifiant lisible par machine (26) est un code à barres présent sur une face extérieure du dispositif médical (6) ou du panier de nettoyage (22) et/ou un OR-code et/ou une étiquette RFID présente sur ou dans l'appareil médical (6) ou le panier de nettoyage (22).

5. Système de traitement (4) selon l'une des revendications 1 à 4, **caractérisé en ce qu'**il comprend une pluralité d'appareils de traitement (14a, 14b, 14c), un dispositif central de commande (44) et un poste de chargement (24), le dispositif central de commande (44) et le poste de chargement (24) étant reliés par des liaisons de données à tous les appareils de traitement (14a, 14b, 14c), et dans lequel le poste de chargement (24) est agencé pour placer l'appareil médical (6) dans le panier de nettoyage (22) et comprend un autre capteur (46) fonctionnant sans contact, qui est conçu pour coopérer avec l'identifiant (26) lisible par machine du dispositif médical (6) et/ou du panier de nettoyage (22) et pour détecter en outre si l'appareil médical (6) et/ou le panier de nettoyage (22) se trouvent au poste de chargement (24) et, si cela est le cas, pour émettre un signal d'occupation au dispositif central de commande (44) du système de traitement (4), le dispositif central de commande (44) attribuant, en réponse à la réception du signal d'occupation, un appareil de traitement disponible (14a, 14b, 14c) au dispositif médical (6) et/ou au panier de nettoyage (22) détecté se trouvant au poste de chargement (24).

6. Système de traitement (4) selon la revendication 5, **caractérisé en ce que** ledit autre capteur (46) est en outre agencé pour détecter si l'appareil médical (6) et/ou le panier de nettoyage (22) sont retirés du poste de chargement (24) et, si c'est le cas, pour émettre un autre signal d'ouverture de porte vers le dispositif central de commande (44), lequel est conçu pour transmettre celui-ci à l'appareil de traitement associé (14a, 14b, 14c), le dispositif de commande (38) de l'appareil de traitement associé (14a, 14b, 14c) étant conçu, en réaction à la réception de cet autre signal d'ouverture de porte, pour permettre l'accès à la salle de traitement par l'ouverture de la porte (16a, 16b, 16c).

7. Système de traitement (4) selon l'une des revendications 1 à 6, **caractérisé en ce que** l'appareil de traitement (14a, 14b, 14c) comporte une première porte (16a, 16b, 16c) et une deuxième porte (18a, 18b, 18c) et l'espace de traitement est fermé ou apte à être fermé sur des côtés opposés par les portes (16a, 16b, 16c, 18a, 18b, 18c) présentes sur des façades d'appareil opposées de l'appareil de traitement (14a, 14b, 14c), la façade d'appareil étant une première façade d'appareil (30a), qui comprend la première porte (16a, 16b, 16c) et étant un côté impur de l'appareil de traitement (14a, 14b, 14c), une deuxième façade d'appareil, qui comprend la deuxième porte (18a, 18b, 18c), étant un côté propre de l'appareil de traitement (14a, 14b, 14c), et le dispositif de commande (38) de l'appareil de traitement (14a, 14b, 14c) étant agencé de façon à permettre, en réponse à la réception du signal d'ouverture de porte, l'accès à l'espace de traitement depuis le côté impur en ouvrant la première porte (16a, 16b, 16c).

8. Procédé d'exploitation d'un système de traitement (4) selon l'une des revendications 1 à 7, **caractérisé en ce que** le capteur (28) détecte en fonction du temps des coordonnées de lieu de l'appareil médical (6) et/ou du panier de nettoyage (22) et les délivre au dispositif de commande (38), le dispositif de commande (38) effectuant une analyse de mouvement de l'appareil médical (6) et/ou du panier de nettoyage (22) en utilisant les coordonnées de lieu dépendant du temps et constatant si l'appareil médical (6) et/ou le panier de nettoyage (22) se déplacent vers la porte (16a, 16b, 16c) de l'appareil de traitement (14a, 14b, 14c) présente sur la façade avant (30a) de l'appareil, et, si le dispositif de commande (38) constate ceci, générant un signal d'ouverture de porte et permettant l'accès à la salle de traitement par l'ouverture de la porte (16a, 16b, 16c), le dispositif de commande (38) déterminant, à partir des coordonnées de lieu dépendant du temps, une courbe spatiale de l'appareil médical (6) et/ou du panier de nettoyage (22) et, si la courbe spatiale se déplace sur au moins une distance prédéfinie et/ou pendant au moins un laps de temps prédéfini et/ou à au moins une vitesse prédéfinie en direction de la porte (16a, 16b, 16c) située sur la façade de l'appareil de traitement (14a, 14b, 14c), cela est considéré comme une indication que l'appareil médical (6) et/ou le panier de nettoyage (22) se déplacent vers la porte (16a, 16b, 16c) de l'appareil de traitement (14a, 14b, 14c).

9. Procédé selon la revendication 8, **caractérisé en ce que** le dispositif de commande (38) détermine, à partir des coordonnées de localisation en fonction du temps, si l'appareil médical (6) et/ou le panier de nettoyage (22) se trouvent à l'intérieur d'une zone de chargement (42a, 42b, 42c) adjacente à la façade (30a) et, si c'est le cas, l'évalue comme étant une indication que l'appareil médical (6) et/ou le panier de nettoyage (22) se déplacent vers la porte (16a, 16b, 16c) de l'appareil de traitement (14a, 14b, 14c).

10. Procédé selon l'une des revendications 8 à 9, **caractérisé en ce que** le système de traitement (4) comprend une pluralité d'appareils de traitement (14a, 14b, 14c), un dispositif central de commande (44) et un poste de chargement (24), le dispositif central de commande (44) et le poste de chargement (24) étant reliés à tous les appareils de traitement (14a, 14b, 14c) par des liaisons de données, le poste de chargement (24) comprenant un autre capteur (46) fonctionnant sans contact, et ledit autre capteur (46) fonctionnant sans contact permettant de détecter si l'appareil médical (6) et/ou le panier de nettoyage (22) se trouvent au niveau du poste de chargement (24) et, si cela est le cas, ledit autre capteur (46) émet un signal d'occupation vers le dispositif central de commande (44) du système de traitement (4), le dispositif central de commande (44) recevant ce signal et, en réaction à la réception de ce signal, attribuant à l'appareil médical (6) et/ou au panier de nettoyage (22) détecté et se trouvant au poste de chargement (24) un appareil de traitement (14a, 14b, 14c) disponible.

11. Procédé selon la revendication 10, **caractérisé en ce que** ledit autre capteur (46) détecte en outre si l'appareil médical (6) et/ou le panier de nettoyage (22) sont retirés du poste de chargement (24) et, si cela est le cas, émet un autre signal d'ouverture de porte vers le dispositif central de commande (44), le dispositif central de commande (44) transmettant cet autre signal d'ouverture de porte à l'appareil de traitement associé (14a, 14b, 14c), et le dispositif de commande (38) de l'appareil de traitement associé (14a, 14b, 14c) permettant, en réaction à la réception de cet autre signal d'ouverture de porte, l'accès à la salle de traitement par ouverture de la porte (16a, 16b, 16c).
